# EUROPÄISCHE PATENTSCHRIFT

(11) **EP 0 799 831 B1**
(45) Veröffentlichungstag und Bekanntmachung des Hinweises auf die Patenterteilung: **24.05.2000**
(21) Anmeldenummer: 97104833.5
(22) Anmeldetag: 21.03.1997
(51) Int. Cl.: C07D 513/22, C09B 47/20

(54) **IR-absorbierende Phthalocyanine**
IR absorbing phthalocyanines
Phtalocyanines absorbant le rayonnement IR

(30) Priorität: 02.04.1996 DE 19613139
(43) Veröffentlichungstag der Anmeldung: 08.10.1997
(73) Patentinhaber: BASF AKTIENGESELLSCHAFT, 67056 Ludwigshafen (DE)
(72) Erfinder: Hagen, Helmut, Dr., 67227 Frankenthal (DE); Albert, Bernhard, Dr., 67147 Forst (DE)

(56) Entgegenhaltungen:
- EP-A- 0 155 780
- EP-A- 0 282 181
- EP-A- 0 305 938
- EP-A- 0 373 643
- EP-A- 0 523 959
- EP-A- 0 607 031
- EP-A- 0 718 375
- PATENT ABSTRACTS OF JAPAN vol. 13, no. 84 (C-572) [3432] , 27.Februar 1989 & JP 63 270765 A (MITSUI TOATSU CHEM INC), 8.November 1988,

## Beschreibung

Die Erfindung betrifft Verbindungen der allgemeinen Formel I 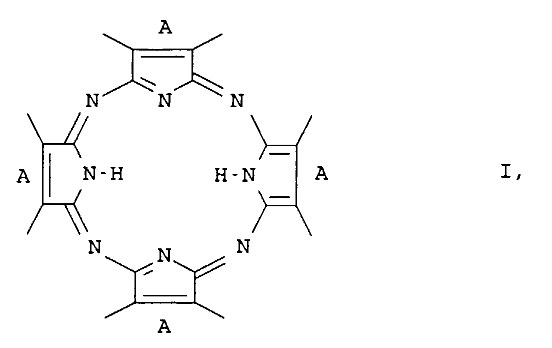 in der die Reste
- A: unabhängig voneinander 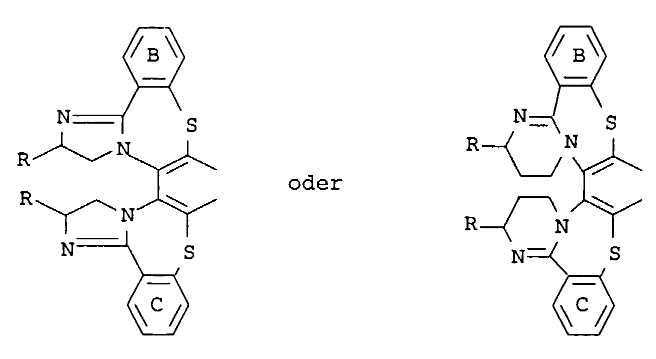 bedeuten, wobei die Ringe
- B und C: unabhängig voneinander noch durch C₁- bis C₄-Alkyl, C₁- bis C₄-Alkoxy, Nitro, Hydroxysulfonyl, Benzoyl oder einen annellierten Benzring substituiert sein können,
- R: Wasserstoff oder Methyl ist und die beiden Wasserstoffatome der Stickstoffe durch Kupfer, Nickel, Vanadyl, Magnesium, AlCl oder Zink ersetzt sein können.

Vorzugsweise ist R Wasserstoff. Weiterhin bevorzugt sind unsubstituierte Ringe B und C oder durch Nitro, Methoxy oder Hydroxysulfonyl substituierte sowie benzannellierte Ringe.

Bevorzugte Zentralatome für die erfindungsgemäßen Phthalocyanine sind Cu und Ni.

Zur Herstellung der Verbindungen der Formel I kann man Verbindungen der Formeln IIa und/oder IIb 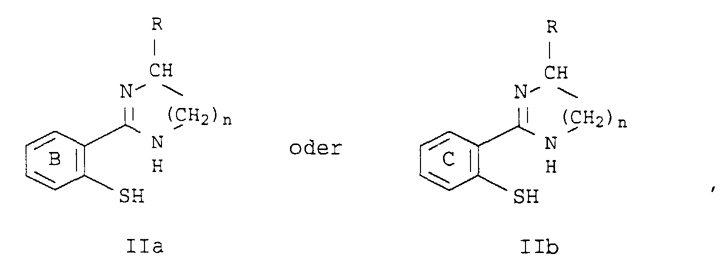 in denen n unabhängig voneinander 1 oder 2 ist und R die angegebene Bedeutung hat, mit halogensubstituierten (vorzugsweise 16 Halogenatome) Phthalocyaninen umsetzen. Einzelheiten der Reaktion können den Beispielen entnommen werden, in denen sich Angaben über Teile und Prozente, sofern nicht anders vermerkt, auf das Gewicht beziehen.

Aus der europäischen Patentschrift 155 780 sind schon Phthalocyanine bekannt, die Schwefel- und/oder Stickstoffsubstituenten tragen; sie werden durch Umsetzung halogensubstituierter Phthalocyanine mit Thiophenolen oder Aminothiophenolen erhalten.

Es ist überraschend, daß sich auch Verbindungen der Formeln IIa und IIb mit Halogenphthalocyaninen umsetzen lassen und zu Phthalocyaninen führen, die in einem Wellenlängenbereich von 720 bis 1100 µm absorbieren.

Aufgrund der sehr effektiven Absorption eignen sich die erfindungsgemäßen Verbindungen für alle Zwecke bei denen eine solche Absorption erwünscht ist, beispielsweise in IR-absorbierenden Tinten, Druckfarben, Kunststoffen (z.B. Brillen oder Agrarfolien, folienkaschierten Scheiben und Fenstern) oder als Absorber beim Laser-Transfer von Farbstoffen im Thermotransferdruck.

Weiterhin können damit IR-lesbare Barcodes oder Markierungen bei Geldscheinen und Wertpapieren hergestellt werden, die als Sicherungssystem fungieren.

### Beispiel 1

In 100 ml N-Methylpyrrolidon werden 35,6 g (0,2 Mol) 2-Mercaptophenylimidazolin und 13,5 g pulverisietes Kaliumhydroxid 10 Minuten bei 120°C gerührt. Dann werden bei gleicher Temperatur 11,5 g Cu-phthalocyanin substituiert mit 15-16 Chloratomen zugegeben und es wird weitere 20 Stunden bei 130°C gerührt. Anschließend wird abgekühlt, vom ausgeschiedenen Salz (KCl und wenig CuCl) abfiltriert und das Lösungsmittel im Vakuum abdestilliert. Der Destillationsrückstand wird in heißem Alkohol gelöst, filtriert und mit Essigester versetzt. Nach dem Abkühlen erhält man 17 g einer graubraunen Verbindung, der nach Elementaranalyse und spektroskopischen Daten die unten angegebene Formel zukommt.

Die Ausbeute entspricht 86 % der Theorie bez. auf Cu-Phthalocyanin.

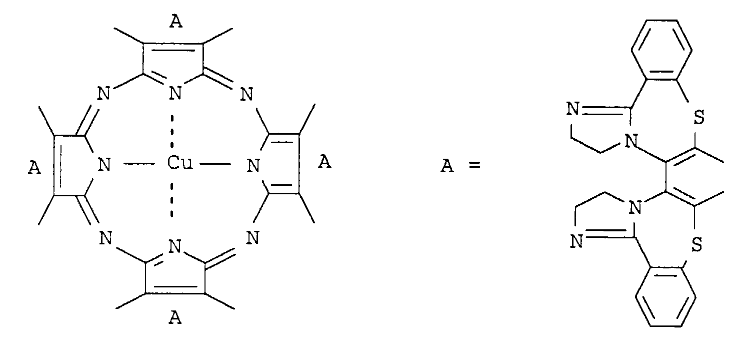

### Beispiel 2

In 100 ml N-Methylpyrrolidon werden 38,4 g (0,2 Mol) 2-Mercaptophenyl-3,4,5,6-tetrahydropyrimidin und 13,5 g pulverisiertes Kaliumhydroxid 30 Minuten bei 120°C gerührt. Nach der Zugabe von 11,5 g des perchlorierten Cu-phthalocyanins wird noch 20 Stunden bei 120° - 130°C gerührt. Nach dem Abkühlen wird filtriert und das Lösungsmittel im Vakuum entfernt. Der Destillationsrückstand wird in heißem Alkohol gelöst, filtriert, mit Essigester versetzt und abgekühlt. Der Niederschlag wird abgesaugt und mit Wasser gewaschen. Nach dem Trocknen erhält man 16,5 g (79 % d. Th.) eines grünschwarzen Cu-phthalocyanins mit der Formel

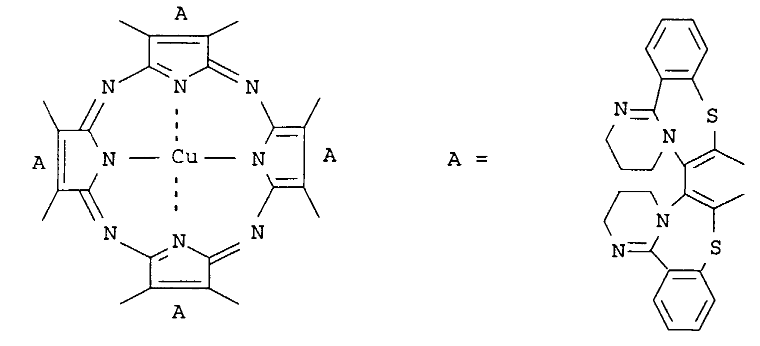

Verwendet man bei der Umsetzung des perhalogenierten Cu-Phthalocyanins ein Gemisch von Verbindungen der Formeln IIa (n = 1, R = H) und IIb (n=2, R=H), wobei die Ringe B und/oder C nicht weiter substituiert sind, so erhält man ein Produkt mit ähnlichen Eigenschaften.

### Anwendungsbeispiel

5 g der Verbindung aus Beispiel 1 wurden in 100 ml Ethanol und 15 g Polyvinylacetat mit 50 g Glaskugeln 2 Stunden auf einer Machine geschüttelt.

Die Mischung wurde auf einer Polyesterfolie aufgerakelt und ergab nach dem Trocknen eine homogene Schicht, die ein Absorptionsmaximum von 780 nm aufwies.

## Patentansprüche

1. Verbindungen der allgemeinen Formel I 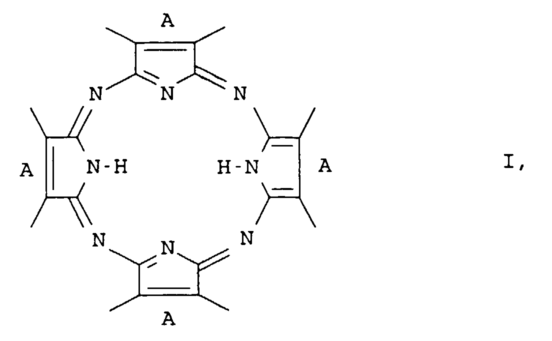 in der die Reste
A unabhängig voneinander 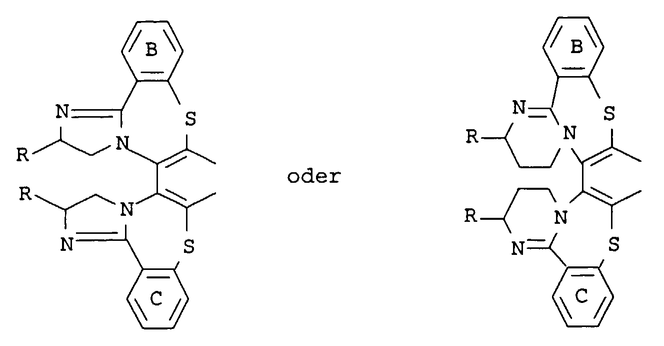 bedeuten, wobei die Ringe
B und C unabhängig voneinander noch durch C₁- bis C₄-Alkyl, C₁- bis C₄-Alkoxy, Nitro, Hydroxysulfonyl, Benzoyl oder einen anellierten Benzring substituiert sein können,
R Wasserstoff oder Methyl ist und die beiden Wasserstoffatome der Stickstoffe durch Kupfer, Nickel, Vanadyl, Magnesium, AlCl oder Zink ersetzt sein können.

2. Verbindungen nach Anspruch 1, bei denen R Wasserstoff ist.

3. Verbindungen nach Anspruch 1, bei denen die Ringe B und C durch Nitro, Methoxy oder Hydroxysulfonyl substituiert, benzanelliert oder unsubstituiert sind.

4. Verbindungen nach Anspruch 1, bei denen das Zentralatom Cu oder Ni ist.

5. Verwendung der Verbindungen nach Anspruch 1 zur IR-Absorption.

## Claims

1. A compound of the formula I 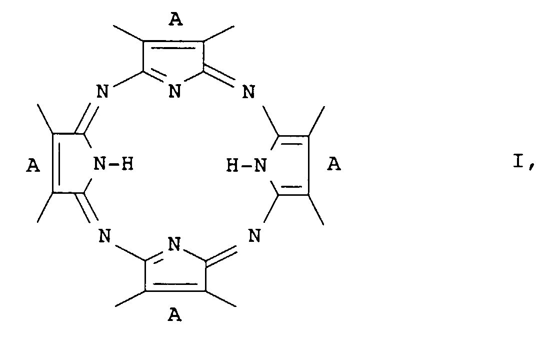 where
the A radicals are, independently of one another, 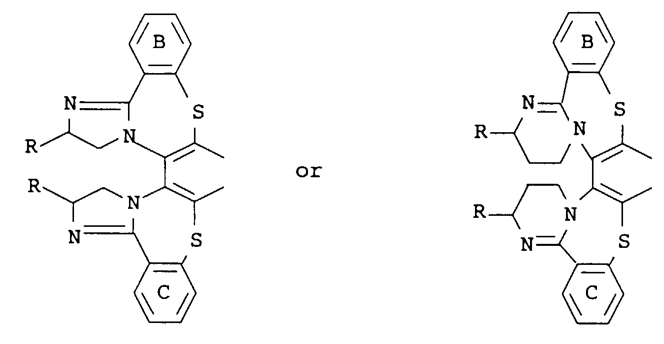 where the rings
B and C can also, independently of one another, be substituted by C₁-C₄-alkyl, C₁-C₄-alkoxy, nitro, hydroxysulfonyl, benzoyl or a fused-on benzo ring,
R is hydrogen or methyl, and the two hydrogen atoms on the nitrogens can be replaced by copper, nickel, vanadyl, magnesium, AlCl or zinc.

2. A compound as claimed in claim 1, where R is hydrogen.

3. A compound as claimed in claim 1, where the rings B and C are substituted by nitro, methoxy or hydroxysulfonyl, are benzo-fused or are unsubstituted.

4. A compound as claimed in claim 1, where the central atom is Cu or Ni.

5. The use of a compound as claimed in claim 1 for IR absorption.

## Revendications

1. Composés de formule générale I 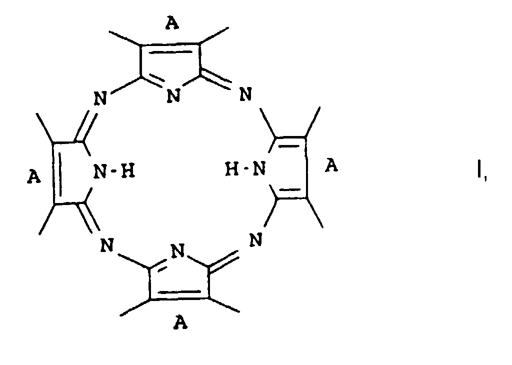 dans laquelle les restes
A représentent indépendamment les uns des autres, 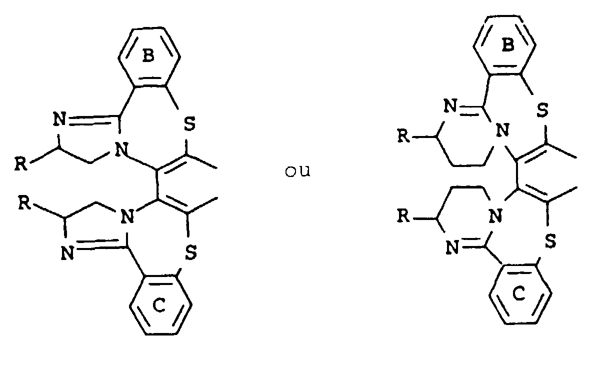 où les cycles
B et C peuvent encore être substitués indépendamment les uns des autres, par des groupes alkyle en C₁ à C₄, alcoxy en C₁ à C₄, nitro, hydroxysulfonyle, benzoyle ou par un noyau benzénique condensé,
R est un atome d'hydrogène ou un groupe méthyle et les deux atomes d'hydrogène des atomes d'azote peuvent être remplacés chacun par un atome de cuivre, un atome de nickel, un groupe vanadyle, un atome de magnésium, AICI ou un atome de zinc.

2. Composés selon la revendication 1, dans lesquels R est un atome d'hydrogène.

3. Composés selon la revendication 1, dans lesquels les cycles B et C sont substitués par des groupes nitro, méthoxy ou hydroxysulfonyle, benzo-condensés ou non substitués.

4. Composés selon la revendication 1, dans lesquels l'atome central est Cu ou Ni.

5. Utilisation des composés selon la revendication 1, pour l'absorption IR.
